# EUROPÄISCHE PATENTSCHRIFT

(11) **EP 0 333 134 B1**
(45) Veröffentlichungstag und Bekanntmachung des Hinweises auf die Patenterteilung: **07.08.1996**
(21) Anmeldenummer: 89104516.3
(22) Anmeldetag: 14.03.1989
(51) Int. Cl.: C12N 15/12, C12P 21/02, C07K 2/00, C12P 21/00, A61K 38/00, C12Q 1/68

(54) **Gentechnische Herstellung von Protein PP 15.**
Gentechnological preparation of protein PP 15.
Méthode de préparation par génie génétique du proteine PP 15.

(30) Priorität: 18.03.1988 DE 3809119
(43) Veröffentlichungstag der Anmeldung: 20.09.1989
(73) Patentinhaber: BEHRINGWERKE Aktiengesellschaft, 35001 Marburg (DE)
(72) Erfinder: Grundmann, Ulrich, Dr., D-3551 Lahntal-Grossfelden (DE); Abel, Karl-Josef, Dr., D-3550 Marburg (DE); Amann, Egon, Dr., D-3550 Marburg (DE)

(56) Entgegenhaltungen:
- EP-A- 0 033 000
- CHEMICAL ABSTRACTS, Band 94, Nr. 15, 13 April 1981, Columbus, OH (US); H. BOHN et al., Seiten 512-513, AN 119251p
- NUCLEIC ACIDS RESEARCH, Band 16, Nr. 10, 25. Mai 1988; U. GRUNDMANN et al., Seite 4721
- BIOCHEMISTRY, Band 18, 1979; J.M. CHIRGWIN et al., Seiten 5294-5299
- ARCHIVES OF GYNECOLOGY, Band 230, 1980; BOHN et al., Seiten 167-172
- BOHN et al., "Immunology of Human Placental Proteins", A. Klopper (Herausg.), Praeger Publishers, 1982, New York, NY (US), Teil 4; Seiten 67-81

## Beschreibung

Das immunsuppressiv wirkende Protein PP 15 ist in der DE-A 29 52 792 (US-A 4,348,316) mit folgenden Parametern beschrieben:
a) einem Kohlenhydratanteil von 3,35 ± 0,9 %, bestehend aus 2,8 ± 0,5 % Hexosen, 0,3 ± 0,2 % Hexosaminen, 0,05 ± 0,05 Fucose und 0,20 ± 0,15 % Neuraminsäure;
b) einem Sedimentationskoeffizienten S_{20.w}⁰ von 2,9 ± 0,2 S;
c) einem in der Ultrazentrifuge bestimmtes Molekulargewicht von 30 700 ± 3 200 (Dimer);
d) einem Extinktionskoeffizienten E_{1cm}^{1 %} (280 nm) von 14,2 ± 1,0 und
e) einer elektrophoretischen Beweglichkeit im Bereich des Albumins sowie
f) einem isoelektrischen Punkt von 4,4 ± 0,1;
g) der Aminosäurezusammensetzung

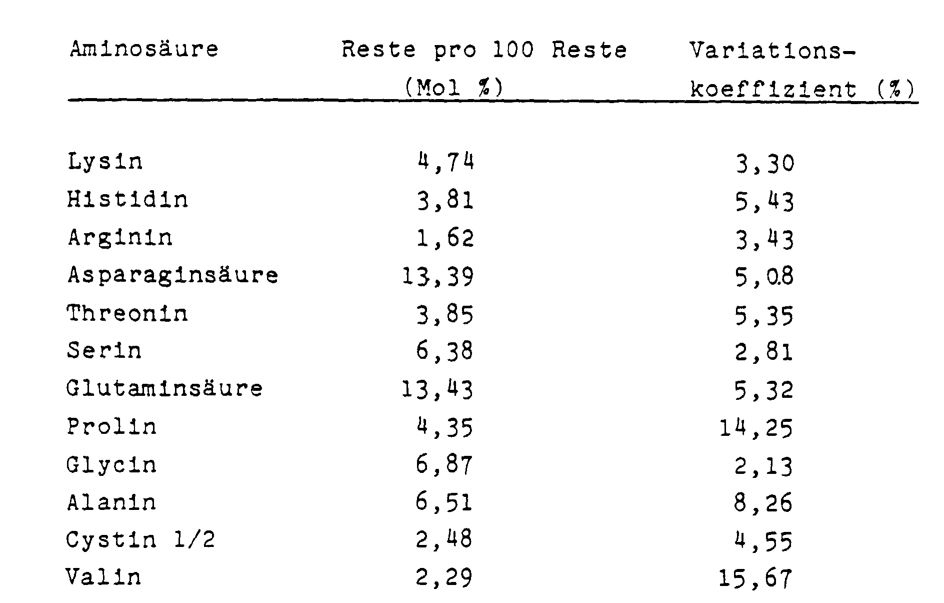

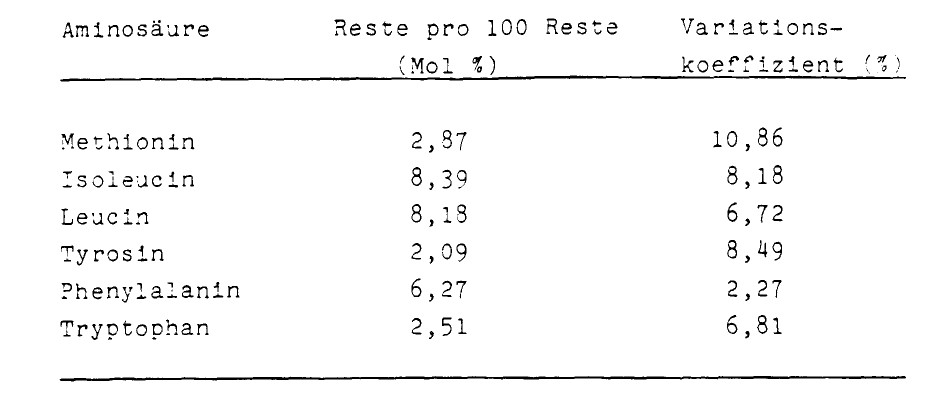

Die Molekulargewichtsbestimmung mittels SDS-Polyacrylamid-Gelelektrophorese ergab ein Molekulargewicht von etwa 15000 d (Monomer).

Wegen des durch die immunsuppressiven Eigenschaften hervorgerufenen therapeutischen und des diagnostischen Interesses ist eine gentechnische Herstellung dieses Proteins äußerst wünschenswert. Die Erfindung betrifft folglich ein Verfahren zur gentechnischen Herstellung von PP15, die dazu erforderliche mRNA, die daraus gewonnene cDNA, diese cDNA ganz oder teilweise enthaltende DNA-Strukturen und Vektoren, mit solcher DNA transformierte Zellen, das von diesen Zellen exprimierte Polypeptid und dessen Verwendung als Arzneimittel. Die Erfindung bezieht sich ferner auf ein immunogenes Polypeptid enthaltend als immunogenen Teil eine Aminosäure - Teilsequenz aus Tabelle 1, welches nicht mit PP15 identisch ist, Teilsequenze damit gewonnene spezifische Antikörper und, aus diesen Antikörpern hergestellte Diagnostika. Eine weitere Ausgestaltung der Erfindung betrifft Diagnostika, die PP15 kodierende bzw. deren komplementäre DNA oder RNA ganz oder teilweise enthalten, und diagnostische Verfahren, mit denen Körperflüssigkeiten und Gewebe mit Hilfe solcher Diagnostika untersucht werden. Weitere Aspekte der Erfindung werden im folgenden näher erläutert bzw. in den Patentansprüchen definiert.

Zunächst wurde versucht, mittels spezifischer Antikörper gegen PP15 in einer kommerziell erhältlichen cDNA-Expressionsbank aus mRNA von reifer humaner Plazenta (Fa. Genofit, Heidelberg) Klone nachzuweisen, die PP15 exprimieren. Da bekannt war, daß PP15 immunsuppressiv wirkt, folglich spezifische Antikörper nur schlecht, wenn überhaupt, herstellbar waren, wurden spezifische Antikörper gegen Teilpeptide hergestellt.

Deshalb wurde das Protein PP15 durch Spaltung mittels Bromcyan, Trypsin oder Proteinase V8 in spezifische Fragmente zerlegt, die anschließend sequenziert wurden. Folgende Fragmente wurden erhalten:
(A) M V V G Q L K A D E D P I M G F H Q M F
(B) F R L A L H N F G
(C) V S V Y A E A A E R
(D) L S S L P F Q K I Q (H)
(E) F D N D R T Q L G A I Y I D A S - L T - E
(F) L L K N I N D A W T

Peptide A, B und C wurden durch allgemein bekannte Methoden synthetisiert und nach üblichen Verfahren in Kaninchen spezifische Antikörper erzeugt. Es gelang nicht, in der o.g. cDNA-Expressionsbank, die ≥ 1 x 10⁶ rekombinierte Lambda gtll Klone enthielt, positive Klone zu lokalisieren. Die Antikörper gegen Peptid A und Peptid B präzipitierten dabei in Kontrollversuchen PP15, während Antikörper gegen Peptid C nicht reagierten. Wie später ersichtlich, ist das Peptid C auch nicht in der nachfolgend aus der cDNA-Sequenz abgeleiteten Proteinsequenz von PP15 enthalten, so daß es wahrscheinlich Begleitproteinen von PP15 zugeordnet werden muß.

Darauf wurden aus den für das PP15-Oligopeptid A kodierenden Oligonukleotiden das PP15-Oligonukleotid-103 5'ATGGTGGTGG GCCAGCTGAA GGCTGATGAG GACCCC, entsprechend aus dem Oligopeptid E das PP15-Oligonukleotid-140 5'TTTGACAATG ACCGGACCCA GCTGGGCGCC ATCTACATTG ATGC und aus dem Oligopeptid F ein 64-fach degeneriertes PP15-Oligonukleotid-139 
5'AAAAATATTAATGATGCCTGGAC
G C C C C
A
 nach statistischen Daten von R. Lathe (J. Mol. Biol. (1985) 183, 1 - 12) ausgewählt.

Mit diesen Oligonukleotidsonden wurde eine cDNA-Bank, die aus mRNA von reifer, humaner Plazenta hergestellt war, einem Screening unterworfen. Zunächst wurde aus der Plazenta die mRNA isoliert und daraus die cDNA hergestellt. Diese wurde mit ECORI-Enden versehen und in die EcoRI-Schnittstelle des Phagenvektors Lambda gt10 ligiert. Es wurden 2 Klone (PP15 - 24 und PP15 - 28) nachgewiesen, die die gesamte cDNA von PP15 enthalten. Die DNA-Sequenzierung erfolgte nach an sich bekannten Methoden; die vollständige Sequenz der PP15 cDNA (kodierender Strang) ist in Tab. 1 gezeigt. Diese cDNA ist 894 Basenpaare (bp) lang, hat am 5'-Ende 99 bp untranslatierter Sequenz, besitzt einen offenen Leserahmen von 381 bp und läßt am 3'-Ende 414 bp einschließlich acht Basen Poly(A) untranslatiert.

In der Tabelle 1 sind die Positionen der Nukleotidsonden durch Unterstreichen markiert, zusätzlich ist die Aminosäuresequenz eingetragen.

Erfindungsgemäß kann die kodierende cDNA mittels geeigneter Expressionssysteme dazu benutzt werden, PP15 zu exprimieren. Durch die Auswahl des Wirtes kann weiterhin die Form der Modifikation von PP15 beeinflußt werden. So findet in Bakterien keine, in Hefezellen eine andere Glykosylierung als in höheren eukaryotischen Zellen statt.

In Kenntnis der Aminosäuresequenz von PP15 ist es möglich, nach konventionellen oder gentechnischen Methoden Aminosäure-Teilsequenzen herzustellen, die als Antigene zur Herstellung von polyklonalen oder monoklonalen Antikörpern dienen können. Solche Antikörper können nicht nur zu diagnostischen Zwecken, sondern auch zur Herstellung von Antikörpersäulen dienen, mit denen PP15 aus Lösungen abgetrennt werden kann, die es neben anderen Proteinen enthalten.

Mit Hilfe der cDNA bzw. Teilen davon kann man auch auf einfache Weise aus einer genomischen Bank den für PP15 codierenden genomischen Klon isolieren, mit dessen Hilfe nicht nur eine Expression in eukaryotischen Zellen erleichtert wird, sondern auch weitere diagnostische Aussagen getroffen werden können.

Die Erfindung ist ferner in den Patentansprüchen definiert und in folgenden Beispielen weiter ausgeführt.

Soweit nicht im Text erläutert, werden die folgenden Abkürzungen verwendet:
- EDTA: = Natrium-ethylendiamin-tetraacetat
- SDS: = Natrium-dodecylsulfat
- DTT: = Dithiothreitol
- BSA: = Rinderserumalbumin

### Beispiele:

### 1. Isolierung von RNA aus humaner Plazenta

RNA wurde aus reifer, humaner Plazenta (nach Chirgwin et al., Biochemistry 18 (1979) 5294-5299) gewonnen. Etwa 10 g Plazentagewebe wurden in flüssigem Stickstoff zermörsert, in 80 ml 4 M Guanidinium-Thiocyanat mit 0,1 M Mercaptoethanol suspendiert und 90 sec. bei 20 000 rpm in einem Homogenisator (Ultraturrax) behandelt. Das Lysat wurde 15 min. bei 7 000 rpm zentrifugiert (Sorvall-GSA Rotor) und der Überstand mit 2 ml 1 M Essigsäure und 60 ml Ethanol abs. bei -20°C über Nacht gefällt. Nach Sedimentation bei 6 000 rpm und -10°C für 10 min. wurden die Nucleinsäuren in 40 ml 7,5 M Guanidinium-Hydrochlorid (pH 7,0) vollständig gelöst und mit einer Mischung aus 1 ml 1 M Essigsäure und 20 ml Ethanol abs. gefällt. Zur Abtrennung der DNA wurde die Fällung ein weiteres Mal mit jeweils halben Volumina wiederholt. Die RNA wurde in 12 ml H₂O gelöst, mit einer Mischung aus 1,2 ml 4 M Kaliumacetat und 24 ml Ethanol abs. gefällt, sedimentiert und schließlich erneut in 10 ml H₂O (1 ml pro g Gewebe) aufgenommen.

### 2. Gewinnung von Poly(A)-haltiger Plazenta-mRNA

Die Plazenta-RNA wurde zur Gewinnung von Poly(A)-haltiger mRNA über Oligo(dT)-Cellulose-Chromatographie (Aviv and Leder, Proc. Natl. Acad. Sci. USA 69 (1973) 1408-1412) in 2 ml Pasteurpipetten In LiCl aufgetrennt. Etwa 5 mg Plazenta-RNA wurden in Puffer 1 (500 mM LiCl, 20 mM Tris (pH 7,5), 1 mM EDTA, 0,1 % SDS) auf die Säule aufgetragen. Während die Poly(A)⁺-RNA an Oligo(dT)-Cellulose gebunden wurde, konnte die Poly(A)⁻-RNA wieder eluiert werden. Nach einem Waschschritt mit Puffer 2 (100 mM LiCl, 20 mM Tris (pH 7,5), 1 mM EDTA, 0,1 % SDS) wurde die Poly(A)⁺-RNA (Plazenta-mRNA) mit Puffer 3 (5 mM Tris (pH 7,5), 1 mM EDTA, 0,05 % SDS) von der Säule eluiert.

Zur weiteren Reinigung wurde die Poly(A)⁺-RNA auf Puffer 1 eingestellt und erneut über Oligo(dT)-Cellulose chromatographiert.

Die Ausbeute an Plazenta Poly(A)⁺-RNA betrug nach diesem zweiten Reinigungsschritt etwa 4 % der eingesetzten RNA.

### 3. Synthese von cDNA aus humaner Plazenta (Plazenta-cDNA) und doppelsträngiger cDNA (dsDNA)

Poly(A)-haltige Plazenta-mRNA wurde vor der cDNA-Synthese im 1,5 % Agarosegel auf Intaktheit geprüft.

Danach wurden 4 µg Plazenta-mRNA in 65,5 µl H₂O gelöst, 10 min. bei 70°C denaturiert und auf Eis gekühlt.

Die cDNA-Synthese erfolgte in einem 100 µl-Ansatz nach Zugabe von 20 µl RT₁-Puffer (250 mM Tris (pH 8,2) bei 42°C, 250 mM KCl, 30 mM MgCl₂), 2,5 µl 20 mM dNTP (d.h. aller vier Desoxynukleosidtriphosphate), 1 µl Oligo(dT) von 1 µg/ml, 1 µl 1 M DTT, 2 µl RNAsin (Boehringer Mannheim) und 8 µl Reverse Transcriptase (24 U/µl, Boehringer Mannheim) für 90 min. bei 42°C. Doppelsträngige cDNA (dsDNA) wurde nach Gubler and Hoffmann (Gene 25 (1983) 263-269) synthetisiert. Die Synthese erfolgte unmittelbar nach der cDNA-Synthese durch Zugabe von 305,5 µl H₂O, 80 µl RT₂-Puffer (100 mM Tris (pH 7,5), 25 mM MgCl₂, 500 mM KCl, 50 mM DTT, 250 µg/ml BSA), 2 µl RNase H (2 U/µl), 2,5 µl E. coli DNA Ligase (5 U/µl), 5 µl 15 mM β-NAD, und 5 µl DNA Polymerase I (5 U/µl) und Inkubation für 5 h bei 15°C. Durch Hitzeinaktivierung (70°C, 30 min.) wurde die Reaktion beendet.

Der Reaktionsansatz wurde nach Zugabe von 55 µl 250 µM dNTP, 55 µl 10 mM Tris (pH 7,5), 10 mM MgCl₂, 10 µg/ml BSA, 3 µl T4 DNA-Polymerase I (1 U/µl), 2 µl RNase H (2 U/µl) und 2 µl RNase A (2 µg/ml) für weitere 30 min. bei 37°C inkubiert, um die Vollständigkeit der Synthese am zweiten DNA-Strang zu gewährleisten ("Repair Reaction").

### 4. Ligierung von EcoRI-Linkern an die dsDNA und Öffnen der Linker

Zur Errichtung einer Plazenta-cDNA-Bank wurde die dsDNA mit EcoRI-Enden versehen, um sie in die EcoRI-Schnittstelle des Phagenvektors λgt10 ligieren zu können (T. Maniatis et al. (1982), Molecular Cloning, A Laboratory Manual, Cold Spring Harbor). Hierzu wurde die dsDNA
a) mit EcoRI-Methylase behandelt, um interne EcoRI-Schnittstellen der dsDNA zu schützen,
b) mit EcoRI-Linkern versehen, die
c) danach mit EcoRI geschnitten wurden.

### Zu a):

Die Methylase-Reaktion der dsDNA erfolgte direkt im Anschluß an die "Repair Reaction" nach Zugabe von 25 µl 500 mM EDTA (pH 8,0), 60 µl Methylase-Puffer (100 mM NaOAc (pH 5,2), 2 mg S-Adenosyl-L-Methionin) und 2 µl EcoRI-Methylase (20 U/µl) durch Inkubation bei 37°C für 30 min.

Der Reaktionsansatz wurde mit Phenol extrahiert und die dsDNA mit 60 µl 4M NaOAc und 1300 µl Ethanol gefällt. Die dsDNA wurde zweimal mit 70 % Ethanol gewaschen, einmal mit Ether ausgeschüttelt und getrocknet.

### Zu b):

Die EcoRI-methylierte dsDNA wurde in 88 µl H₂O gelöst und nach Zugabe von 10 µl Ligase-Puffer (500 mM Tris (pH 7,4), 100 mM MgCl₂, 100 mM DTT, 10 mM Spermidin, 10 mM ATP, 1 mg/ml BSA), 1 µl T4 DNA-Ligase (10 U/µl) mit 1 µl EcoRI Linkern (0,5 µg/µl) (pGGAATTCC bzw. pAGAATTCT) über Nacht bei 15°C ligiert.

### Zu c):

Das Volumen des Ligase-Ansatzes wurde mit 6 µl H₂O, 12 µl 10x EcoRI-Puffer und 2 µl EcoRI (120 U/µl) auf 120 µl gebracht. Die EcoRI-Verdauung erfolgte für 2 h bei 37°C.

### 5. Abtrennen nichtgebundener Linker über Kaliumacetat-Gradienten und Größenselektionierung der dsDNA

Zur Abtrennung aller nichtgebundenen EcoRI-Linker von der dsDNA wurde der EcoRI-Reaktionsansatz in toto auf einen Kaliumacetat-Gradienten (5-20 % KOAc, 1 mM EDTA, 1 µl/ml Ethidiumbromid) aufgetragen und 3 h bei 50 000 rpm und 20°C zentrifugiert (Beckman SW 65-Rotor).

Der Gradient wurde von unten so fraktioniert, daß die ersten fünf Fraktionen 500 µl maßen und alle restlichen 100 µl. Die Fraktionen wurden mit 0,01 Volumen Acrylamid (2 mg/ml) und 2,5 Volumen Ethanol gefällt, einmal mit 70 %igem Ethanol gewaschen, getrocknet und jeweils in 5 µl H₂O aufgenommen.

Zur Größenbestimmung der dsDNA wurden 1 µl jeder Fraktion im 1,5 % Agarose-Gel analysiert. Zusätzlich wurde mit 1 µl jeder Fraktion die Quantität an dsDNA bestimmt.

Fraktionen mit dsDNA über 500 bp wurden vereinigt und die Probe so eingeengt, daß die Endkonzentration 27 µg/ml betrug.

### 6. Einbau der dsDNA in den Phagenvektor λgt10 und "in vitro packaging"-Reaktion

Der Einbau der dsDNA in die EcoRI-Schnittstelle des Phagenvektors λgt10 (Vector Cloning Systems, San Diego, CA) erfolgte in einem Ligaseansatz von 4 µl: 2 µl dsDNA, 1 µl λgt10 x EcoRI (1 µg/ml), 0,4 µl Ligase-Puffer, 0,5 µl H₂O, 0,1 µl T4 DNA-Ligase. Der Ansatz wurde 4 h bei 15°C inkubiert.

Zur Etablierung der Plazenta-cDNA-Bank im Phagenvektor λgt10 folgte mit den λ-lysogenen Zellextrakten E. coli NS 428 und NS 433 eine "in vitro packaging"-Reaktion des Ligaseansatzes bei Raumtemperatur für 2 h (Vector Cloning Systems, San Diego, CA; Enquist and Sternberg, Methods in Enzymology 68, (1979), 281-298). Die Reaktion wurde mit 500 µl Suspensionsmedium (SM: 0,1 M NaCl, 8 mM MgSO₄, 50 mM Tris (pH 7,5), 0,01 % Gelatine) und 2 Tropfen Chloroform gestoppt.

### 7. Titerbestimmung und Analyse der Plazenta-cDNA-Bank

Die Zahl der "Plaque Forming Units" (PFU) der Plazenta-cDNA-Bank wurde mit kompetenten Zellen des E. coli-K 12-Stammes C600 HFL bestimmt: Sie betrug 1 x 10⁶ PFU.

### 8. Oligonukleotidsonden zum "Screening" der Plazenta-cDNA-Bank

Oligonukleotidsonden (PP15-Oligonukleotid 103 und 140) und ein Pool von Oligonukleotiden (PP15-Oligonukleotidpool 139) wurden zur Analyse der Plazenta-cDNA-Bank synthetisiert. Ihre Sequenzen wurden abgeleitet von der Aminosäuresequenz dreier Bromcyanfragmente von PP15.

Die Art des Aufbaus und die Verwendung der Sonden folgten im wesentlichen den Regeln von R. Lathe, a.a.O..

Die Oligonukleotidsequenzen wurden mit T4 Polynukleotidkinase unter Anwesenheit von (γ-32P)ATP am 5′-Ende markiert (eingesetzt wurden 60 µCi/40 µl Reaktionsansatz). Die Sonden hatten eine spezifische Aktivität von 1 x 10⁸ Bq/µl bzw. 1,5 x 10⁶ Bq/pMol.

### 9. "Screening" der Plazenta-cDNA mit PP15 spezifischen Oligonukleotiden

1 x 10⁶ PFU der Plazenta-cDNA-Bank wurden mit den PP15-Oligonukleotidsonden 103, 140 und 139 zusammen untersucht. Dazu wurden je 3 x 10⁴ PFU mit Zellen des E. coli K 12-Stammes C 600 HFL in Weich-Agar auf 13,5 cm Petrischalen ausplattiert und 6 h bei 37°C inkubiert. Zu diesem Zeitpunkt war noch keine konfluente Lyse eingetreten. Die Platten wurden über Nacht im Kühlschrank inkubiert und die Phagen auf Nitrocellulosefilter (Schleicher & Schüll, BA 85, Ref.-Nr. 401124) übertragen (Duplikate). Nitrocellulosefilter und Petrischalen wurden mit einer Injektionskanüle markiert, um eine spätere Zuordnung positiver Plaques zu ermöglichen. Die Petrischalen wurden während des Prozessierens der Nitrocellulosefilter im Kühlraum gelagert. Die auf den Nitrocellulosefiltern befindliche DNA wurde denaturiert, indem die Filter für 5 min. auf mit 1,5 M NaCl, 0,5 M NaOH-getränktes Filterpapier (Whatman-M3) gelegt wurden. Anschließend wurden die Filter auf die gleiche Art mit 1,5 M NaCl, 0,5 M Tris (pH 8,0) renaturiert und mit 2 x SSPE (0,36 M NaCl, 16 mM NaOH, 20 mM NaH₂PO₄, 2 mM EDTA) gewaschen. Die Filter wurden dann für 2 h bei 80°C im Vakuum getrocknet. Die Filter wurden für 4 h bei 65°C in 3xSSC, 0,1 % SDS (20 x SSC = 3 M NaCl, 0,3 M Na-Citrat) gewaschen und für 4 h bei 65°C vorhybridisiert (Prähybridisierungslösung: 0,6 M NaCl, 0,06 M Tris (pH 8,3), 6 mM EDTA, 0,2 % nichtionisches synthetisches Saccharose-Polymer (^{R}Ficoll), 0,2 % Polyvinylpyrrolidon 40, 0,2 % BSA, 0,1 % SDS, 50 µg/ml denaturierte Heringssperma-DNA). Die Filter wurden über Nacht unter Zusatz von 100 000-200 000 Bq des markierten Oligonukleotids/ml Hybridisierungslösung (wie Prähybridisierungslösung, jedoch ohne Heringssperma-DNA) in Bechergläsern bzw. in zugeschweißten Polyethylenfolien unter leichtem Schütteln inkubiert. Die Hybridisierungstemperatur betrug 46°C für die Oligonukleotidsonde 139 bzw. 52°C für die anderen Sonden. Die Nitrocellulosefilter wurden mit 6 x SSC, 0,05 M Natriumpyrophosphat eine Stunde bei Raumtemperatur und für eine weitere Stunde bei der jeweiligen Hybridisierungstemperatur gewaschen. Die Filter wurden getrocknet und über Nacht autoradiographiert. Signale auf dem Röntgenfilm, die auf beiden Duplikaten auftraten, wurden der Petrischale zugeordnet und die Region (ca. 50 Plaques) mit dem breiten Ende einer Pasteurpipette ausgestanzt und die Phagen in 1 ml SM-Puffer resuspendiert. Positive Phagen wurden über drei Runden vereinzelt, bis ein einzelner postiviter Klon vorlag.

Je 1 x 10⁶ PFU der Plazenta-cDNA-Bank wurden dreimal untersucht. Erst beim dritten Screening wurden 2 Signale auf Duplikat-Filtern identifiziert. Die beiden Klone PP15 - 24 und PP15 - 28 enthalten die gesamte cDNA von PP15.

Der Vergleich der Oligonukleotidsequenzen 103, 139 und 140 mit der ermittelten PP15-Sequenz ist in Tab. 2 zusammengefaßt.

PP15-Sequenz vs. PP15-Oligonukleotid 103

. . . . .

301 ATCACCGCGCAGGACCATCAGCCCACTCCAGATAGCTGCATCATCAGCAT 350
1 ................................................AT 2

. . . . .

351 GGTTGTGGGCCAGCTTAAGGCGGATGAAGACCCCATCATGGGGTTCCACC 400
3 GGTGGTGGGCCAGCTGAAGGCTGATGAGGACCCC................ 36

PP15-Sequenz vs. PP15-Oligonukleotid 139

. . . . .

401 AGATGTTCCTATTAAAGAACATCAACGATGCTTGGGTTTGCACCAATGAC 450
1 ..............AAGAACATCAACGATGCCTGGAC............. 23

PP15-Sequenz vs. PP15-Oligonukleotid 140

. . . . .

151 TACTACCAGTTATTTGATAATGATAGAACCCAACTAGGCGCAATTTACAT 200
1 ............TTTGACAATGACCGGACCCAGCTGGGCGCCATCTACAT 38

. . . . .

201 TGACGCGTCATGCCTTACGTGGAAGGACAACAGTTCCAGGGGAAAGCTG 250
39 TGATGC............................................ 44

### 10. DNA-Sequenzanalyse

Die Phagenklone PP15 - 24 und PP 15 - 28 wurden vermehrt und ihre DNA jeweils extrahiert. Das jeweilige EcoRI-Fragment wurde isoliert und in die EcoRI-Stelle des Bluescript M13 Vektors (Stratagene, San Diego, CA, USA) für Restriktionsanalysen sowie Sequenzanalysen mittels der enzymatischen Dideoxymethode nach Sanger einligiert. Die Sequenz zeigt einen offenen Leserahmen und kodiert für ein Protein mit maximal 127 Aminosäuren. PP15 besitzt ein errechnetes Molekulargewicht von 14478 d (einschließlich Methionin), was mit dem eingangs genannten Wert der Patentschrift DE-A 29 52 792 gut übereinstimmt.

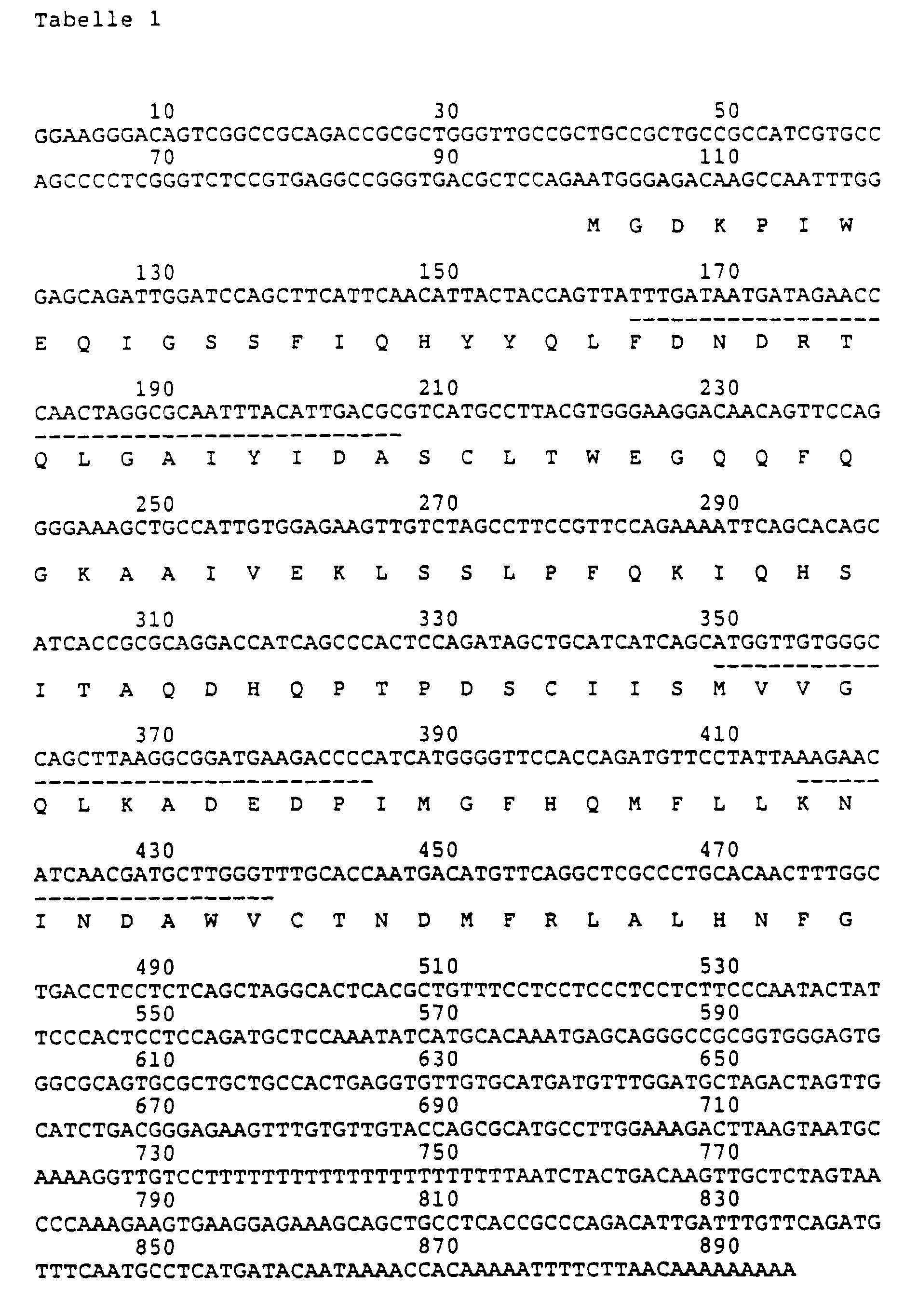

### 11. Expression des immunsuppressiven Protein PP15

Der Vektor pTrc99A (E. Amann et al. (1988) Gene 69, 301-315) wurde zur Expression des unfusionierten, reifen PP15 Proteins in E.coli benutzt. Die DNA Sequenz der PP15 cDNA am Initiationscodon lautet:

                    Met Gly Asp
5'  ... CGCTCCAGA  ATG GGA GAC ...3'

Da keine NcoI stelle am ATG vorhanden ist, kann diese DNA nicht direkt in den pTrc99A Expressionsvektor kloniert werden. Eine NcoI Stelle kann aber durch zwei Basenaustausche in der PP15 Sequenz: 5' GAATGG 3' nach 5' CCATGG 3' durch Mutagenese erzielt werden. Die zweite Aminosäure (Gly) ist von dieser Manipulation nicht betroffen, da das zweite Codon der PP15 Struktursequenz mit einem "G" beginnt. Zur Mutagenese wurde ein 902 Basenpaar großes EcoRI-Fragment des PP15 cDNA Klons PP15-28 isoliert und in das ebenfalls mit EcoRI geschnittenen und dephosphorylierten Mutagenesevektor pMa5-8 (Fig.) ligiert. Das resultierende Plasmid pMa5-8-PP15 (mit der korrekten Orientierung des PP15 EcoRI-Inserts in Relation zum F1-Origo) wurde dann dem "gapped duplex" Mutageneseprotokoll (Kramer et al. (1984) Nucl. Acids. Res. 12, 9441-9456) unterzogen, wobei das folgende Oligodesoxynukleotid verwendet wurde:

5' GGCTTGTCTCCCATGGTGGAGCGTCAC 3'

Ein Klon, der die gewünschte Mutation aufwies, wurde durch Restriktionsanalyse identifiziert und als pMc5-8-PP15-NcoI bezeichnet. Aus diesem Plasmid wurde das 798 Basenpaar große NcoI-EcoRI-Fragment isoliert und in den entsprechenden geschnittenen pTrc99A Vektor ligiert. Das resultierende Plasmid pTrc99A-PP15 umfaßt 4918 Basenpaare und exprimiert nach Induktion des trc Promoters das unfusionierte, ca. 15 kD große PP15 Protein.

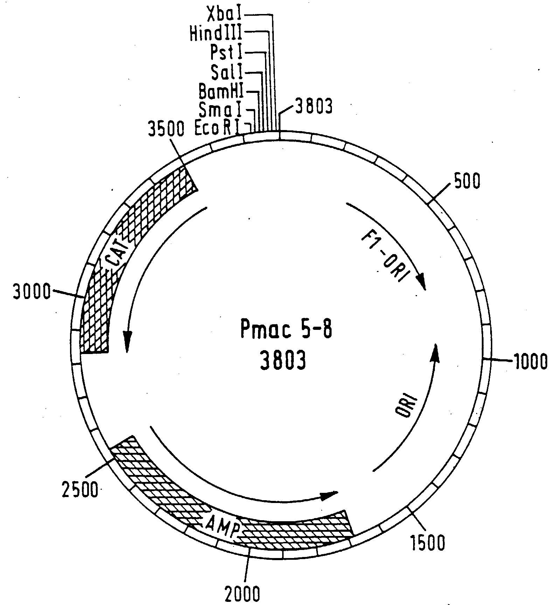

### Legende zu Fig. 1:

Karte der Plasmide pMAC5-8 (= pMA5-8 und pMC5-8).
- F1-ORI:: Replikationsursprung des Phagen f1;
- ORI:: Replikationsursprung vom ColE1-Typ;
- CAT:: Kodierende Region für Chloramphenicol-acetyltransferase;
- AMP:: Kodierende Region für β-Lactamase.
pMA5-8 trägt eine amber-Mutation in CAT (A bei Position 3409) und pMC5-8 eine amber-Mutation in AMP (C bei Position 2238).

## Patentansprüche (Patentansprüche für folgende(n) Vertragsstaat(en): AT, BE, CH, DE, FR, GB, IT, LI, LU, NL, SE)

1. Isolierte DNA, kodierend für ein Polypeptid mit der Aminosäuresequnz gemäß Tabelle 1 oder für immunogene Teile davon.

2. Isolierte DNA oder RNA, die mit der DNA nach Anspruch 1 unter stringenten Bedingungen hybridisiert.

3. Vektoren, enthaltend eine DNA nach Anspruch 1 oder 2.

4. Transformierte Zelle, enthaltend eine DNA nach Anspruch 1 oder 2.

5. Imunogenes Polypeptid, welches als immunogenen Teil eine Aminosäure-Teilsequenz aus Tabelle 1 enthält und welches nicht mit PP15 identisch ist.

6. Gentechnisch durch Expression in Hefe erhältliches Polypeptid gemäß Anspruch 5.

7. Gentechnisch durch Expression in E. coli erhältliches Polypeptid gemäß Anspruch 5.

8. Verfahren zur gentechnischen Herstellung von PP15 oder eines immunogenen Teils davon, dadurch gekennzeichnet, daß man die DNA nach Anspruch 1 in einem geeigneten Wirt zur Expression bringt.

9. Für PP15 spezifische polyklonale oder monoklonale Antikörper, erhalten durch Immunisierung mit dem Polypeptid gemäß Anspruch 5.

10. Diagnostikum, enthaltend eine DNA nach Anspruch 1 oder 2.

11. Diagnostikum, enthaltend einen Antikörper nach Anspruch 9.

12. Diagnostikum, enthaltend das Polypeptid nach Anspruch 5.

13. Arzneimittel, enthaltend das Polypeptid nach Anspruch 5.

## Patentansprüche (Patentansprüche für folgende(n) Vertragsstaat(en): ES, GR)

1. Verfahren zur Herstellung von PP15 oder eines immunogenen Teils davon, dadurch gekennzeichnet, daß man eine für PP15 nach Tabelle 1 kodierende cDNA in ein Expressionssystem einbringt und dort zur Expression bringt.

2. Verfahren nach Anspruch 1, dadurch gekennzeichnet, daß das Expressionsystem ein Eukaryot ist, vorzugsweise Hefe.

3. Verfahren nach Anspruch 1, dadurch gekennzeichnet, daß das Expressionsystem ein Prokaryot ist, vorzugsweise Escherichia coli.

4. Verfahren zur Herstellung von mono- oder polyklonalen Antikörpern gegen PP15, dadurch gekennzeichnet, daß ein nach Anspruch 1, 2 oder 3 hergestellter Teil von PP15 zur Immunisierung verwendet wird.

5. Verfahren zur Herstellung eines Arzneimittels, dadurch gekennzeichnet, daß ein nach Anspruch 1, 2 oder 3 hergestellter immunogener Teil von PP15 mit üblichen Trägern vermischt wird.

6. Verfahren zur Herstellung eines Diagnostikums, dadurch gekennzeichnet, daß ein nach Anspruch 1, 2 oder 3 hergestellter immunogener Teil von PP15 eingesetzt wird.

7. Verfahren zur Herstellung eines Diagnostikums, dadurch gekennzeichnet, daß für PP15 nach Tabelle 1 kodierende cDNA eingesetzt wird.

8. Verfahren zur Herstellung eines Diagnostikums, dadurch gekennzeichnet, daß nach Anspruch 4 hergestellte mono- oder polyklonale Antikörper eingesetzt werden.

## Claims (Claims for the following Contracting State(s): AT, BE, CH, DE, FR, GB, IT, LI, LU, NL, SE)

1. An isolated DNA coding for a polypeptide having the amino acid sequence shown in Table 1, or for immunogenic parts thereof.

2. An isolated DNA or RNA which hybridizes with the DNA as claimed in claim 1 under stringent conditions.

3. A vector containing a DNA as claimed in claim 1 or 2.

4. A transformed cell containing a DNA as claimed in claim 1 or 2.

5. An immunogenic polypeptide which contains as immunogenic part an amino-acid part sequence from Table 1, and which is not identical to PP15.

6. A polypeptide as claimed in claim 5 obtainable by genetic manipulation and by expression in yeast.

7. A polypeptide as claimed in claim 5 obtainable by genetic manipulation and by expression in E. coli.

8. A process for the preparation of PP15 or of an immunogenic part thereof by genetic manipulation, which comprises expressing the DNA as claimed in claim 1 in a suitable host.

9. Polyclonal or monoclonal antibodies specific for PP15, obtained by immunization with the polypeptide as claimed in claim 5.

10. A diagnostic aid containing a DNA as claimed in claim 1 or 2.

11. A diagnostic aid containing an antibody as claimed in claim 9.

12. A diagnostic aid containing a polypeptide as claimed in claim 5.

13. A pharmaceutical containing the polypeptide as claimed in claim 5.

## Claims (Claims for the following Contracting State(s): ES, GR)

1. A process for the preparation of PP15 or of an immunogenic part thereof, which comprises insertion of a cDNA coding for PP15 as shown in Table 1 into an expression system, and bringing about expression therein.

2. The process as claimed in claim 1, wherein the expression system is a eukaryote, preferably yeast.

3. The process as claimed in claim 1, wherein the expression system is a prokaryote, preferably Escherichia coli.

4. A process for the preparation of mono- or polyclonal antibodies against PP15, which comprises using a part, prepared as claimed in claim 1, 2 or 3, of PP15 for immunization.

5. A process for the preparation of a pharmaceutical which comprises mixing an immunogenic part, prepared as claimed in claim 1, 2 or 3, of PP15 with conventional vehicles.

6. A process for the preparation of a diagnostic acid, which comprises using an immunogenic part, prepared as claimed in claim 1, 2 or 3, of PP15.

7. A process for the preparation of a diagnostic aid, which comprises using cDNA coding for PP15 as shown in Table 1.

8. A process for the preparation of a diagnostic aid, which comprises using mono- or polyclonal antibodies prepared as claimed in claim 4.

## Revendications (Revendications pour l'(les) Etat(s) contractant(s) suivant(s): AT, BE, CH, DE, FR, GB, IT, LI, LU, NL, SE)

1. ADN isolé codant pour un polypeptide ayant la séquence d'amino-acides selon le tableau 1 ou pour des parties immunogènes de celui-ci.

2. ADN ou ARN isolé qui s'hybride avec l'ADN selon la revendication 1 dans des conditions sévères.

3. Vecteurs contenant un ADN selon la revendication 1 ou 2.

4. Cellule transformée contenant un ADN selon la revendication 1 ou 2.

5. Polypeptide immunogène qui contient, comme partie immunogène, une séquence partielle d'amino-acides du tableau 1 et qui n'est pas identique à la PP15.

6. Polypeptide selon la revendication 5, qu'on peut obtenir par génie génétique, par expression dans une levure.

7. Polypeptide selon la revendication 5, qu'on peut obtenir par génie génétique, par expression dans *E. coli.*

8. Procédé de préparation, par génie génétique, de la PP15 ou d'une partie immunogène de celle-ci, caractérisé en ce qu'on exprime l'ADN selon la revendication 1 dans un hôte approprié.

9. Anticorps polyclonaux ou monoclonaux spécifiques de la PP15, qu'on obtient grâce à une immunisation avec le polypeptide selon la revendication 5.

10. Agent de diagnostic contenant un ADN selon la revendication 1 ou 2.

11. Agent de diagnostic contenant un anticorps selon la revendication 9.

12. Agent de diagnostic contenant le polypeptide selon la revendication 5.

13. Médicament contenant le polypeptide selon la revendication 5.

## Revendications (Revendications pour l'(les) Etat(s) contractant(s) suivant(s): ES, GR)

1. Procédé de préparation de PP15 ou d'une partie immunogène de celle-ci, caractérisé en ce qu'on introduit un ADNc codant pour la PP15, selon le tableau 1, dans un système d'expression et on l'exprime dans celui-ci.

2. Procédé selon la revendication 1, caractérisé en ce que le système d'expression est un eucaryote, de préférence une levure.

3. Procédé selon la revendication 1, caractérisé en ce que le système d'expression est un procaryote, de préférence *Escherichia coli.*

4. Procédé de préparation d'anticorps monoclonaux ou polyclonaux dirigés contre la PP15, caractérisé en ce qu'on utilise une partie de la PP15, préparée selon la revendication 1, 2 ou 3, pour l'immunisation.

5. Procédé de préparation d'un médicament, caractérisé en ce qu'on mélange une partie immunogène de la PP15, préparée selon la revendication 1, 2 ou 3, avec des véhicules usuels.

6. Procédé de préparation d'un agent de diagnostic, caractérisé en ce qu'on utilise une partie immunogène de la PP15 préparée selon la revendication 1, 2 ou 3.

7. Procédé de préparation d'un agent de diagnostic, caractérisé en ce qu'on utilise un ADNc codant pour la PP15, selon le tableau 1.

8. Procédé de préparation d'un agent de diagnostic, caractérisé en ce qu'on utilise des anticorps monoclonaux ou polyclonaux préparés selon la revendication 4.
